# EUROPEAN PATENT SPECIFICATION

(11) **EP 0 828 705 B1**
(45) Date of publication and mention of the grant of the patent: **23.08.2000**
(21) Application number: 96914332.0
(22) Date of filing: 21.05.1996
(51) Int. Cl.: C07C 231/12, C07C 237/46

(54) **IODINATION PROCESS**
VERFAHREN ZUR IODIERUNG
PROCEDE D'IODATION

(30) Priority: 24.05.1995 US 449272; 06.06.1995 GB 9511367
(43) Date of publication of application: 18.03.1998
(73) Proprietor: NYCOMED IMAGING AS, 0401 Oslo 4 (NO)
(72) Inventor: WISTRAND, Lars-Göran, N-0401 Oslo (NO); GOLMAN, Klaes, N-0401 Oslo (NO); RADNER, Finn, N-0401 Oslo (NO)
(74) Representative: Skailes, Humphrey John
(86) International application number: GB9601219
(87) International publication number: WO9637461

(56) References cited:
- US-A- 5 013 865
- TETRAHEDRON LETT. (1989), 30(13), 1649-50 CODEN: TELEAY;ISSN: 0040-4039, 1989, XP000572794 SHONO, TATSUYA ET AL: "Electroorganic chemistry. 117. Aromatic iodination by positive iodine active species generated by anodic oxidation in trimethyl orthoformate" cited in the application
- J. AM. CHEM. SOC. (1976), 98(6), 1515-19 CODEN: JACSAT, 1976, XP000573922 MILLER, LARRY L. ET AL: "Scope and mechanism of aromatic iodination with electrochemically generated iodine(I)"

## Description

The present invention relates to a process for the production of monomeric and dimeric triiodophenyl compounds by electrochemical iodination.

### BACKGROUND OF THE INVENTION

In diagnostic X-ray imaging, iodine compounds are frequently used as contrast enhancing agents. The compounds used for this purpose for the most part are compounds containing one or two triiodophenyl groups, and thus are often referred to respectively as monomers and dimers.

Examples of commercially available triiodophenyl monomers and dimers include the monomeric compounds acetrizoate, diatrizoate, diodone, iobenzamate, iocetamate, iodamide, iodohippurate, ioglicate, iopamoate, iophendylate, iopronate, iothalamate, ioxitalamate, ipodate, metrizoate, iomeprol, iopentol, iopromide, iosimide, ioversol, ioglucol, iogluamide, ioglunide, iogulamide, iosarcol, ioxilan, metrizamide, iopamidol and iohexol, and the dimeric compounds iocarmate, iodipamide, iodoxamate, ioglycamate, ioxaglate, iotroxate, iotasul, iotrolan, iodecimol and iodixanol.

Other monomers and dimers are known from the literature and may be prepared in accordance with the present invention, for example the monomers and dimers referred to in WO-94/14478 (Bracco).

In the conventional processes used for the preparation of these compounds, iodination of the aromatic rings is generally the last or one of the last process steps as in this way utilization of the relatively expensive iodinating agents is optimised.

Thus, for example, in the production of iohexol or iodixanol, the iodination step is conventionally effected using the intermediate 3,5-bis(2,3-dihydroxypropylaminocarbonyl)-aniline.

The iodination reaction, serving to introduce iodines at all of the unsubstituted aromatic ring positions, is conventionally performed using ICl (or its iodide salt analogues such as KICl₂ or NaICl₂) in an acidic aqueous medium. Examples of such iodination reactions can be found for example in WO-92/14695 (Guerbet) and US-A-5013865 (Mallinckrodt).

These ICl iodination agents are commercially available as corrosive aqueous solutions which have a limited storage life. Furthermore, in the iodination reaction some chlorination may occur as an undesired side-reaction.

Mono-iodination of aromatic species by a procedure known as electrochemical iodination, has been described for example by Shono et al in Tetrahedran Letters 30:1649-1650 (1989). This procedure involves anodic generation from an iodine source of iodide (I⁺) cations which react to substitute the aromatic ring of a target aromatic compound.

Shono et al (supra) and the other investigators of electrochemical iodination have only suggested its use for introducing a single iodine onto the aromatic ring of the target compound, generally with high para-position specificity.

It has however now been found that triiodination of the monomeric intermediates and hexaiodination of the dimeric intermediates for the iodinated X-ray contrast agents can be effected in high yield by electrochemical iodination in an acidic solvent mixture which comprises water and optionally at least one water-miscible organic solvent, preferably a polar and/or protic solvent. This route allows the use of cheaper, non-corrosive and highly stable iodine sources (such as iodine and potassium iodide for example) and thus offers significant benefits in the commercial production of iodinated X-ray contrast agents.

### SUMMARY OF THE INVENTION

Thus, viewed from one aspect, the present invention provides a process for the preparation of a 2,4,6-triiodinated or 2,4,6,2',4',6'-hexaiodinated 3,5-disubstituted-aniline or 3,3'-disubstituted-5,5'-linked bisaniline, which process comprises electrochemically iodinating a 3,5-disubstituted-aniline or a 3,3'-disubstituted-5,5'-linked bisaniline in an aqueous acidic solvent, eg. a solvent which comprises water and optionally at least one water-miscible organic solvent.

### DETAILED DESCRIPTION OF THE INVENTION

In one embodiment, the process of the invention is a process for the preparation of compounds of formula I 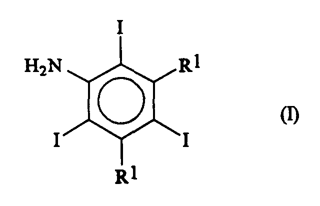 (wherein R¹ is as defined below and one R¹ group may represent a group of formula II 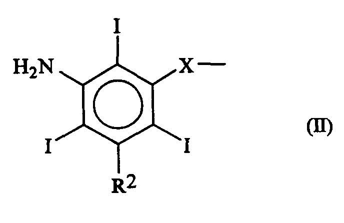 where R² is as defined for R¹ with the exception of the group of formula II and X is a bond or a moiety providing a 1-7 atom chain linking the aromatic rings) by electrochemically iodinating a compound of formula III 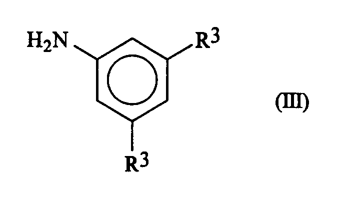 (wherein R³ is as defined below and one R³ group may be a group of formula IV 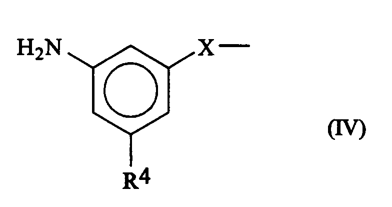 where R⁴ is as described for R³ with the exception of the group of formula IV).

The substituent groups R¹, R², R³, and R⁴ may be any of the ionizing or more preferably non-ionizing groups conventionally used to enhance water solubility. Suitable groups include for example straight chain or branched C₁₋₈-alkyl groups, preferably C₁₋₅ groups, optionally with one or more CH₂ or CH moieties replaced by oxygen or nitrogen atoms and optionally substituted by one or more groups selected from oxo, hydroxy, alkoxy, amino, carboxyl, carbonyl derivative, and oxo substituted sulphur and phosphorus atoms. Particular examples include polyhydroxyalkyl, hydroxyalkoxyalkyl and hydroxypolyalkoxyalkyl and such groups attached to the phenyl group via an amide, CO, SO or SO₂ linkage such as hydroxyalkylaminocarbonyl, N-alkyl-hydroxyalkylaminocarbonyl and bis-hydroxyalkylaminocarbonyl groups as well as COCH₂OH and SO₂CH₂OH. Preferred among such groups are those containing 1, 2, 3, 4, 5 or 6, especially 1, 2 or 3, hydroxy groups, e.g.
-CONH-CH₂CH₂OH
-CONH-CH₂CHOHCH₂OH
-CONH-CH(CH₂OH)₂
-CON(CH₂CH₂OH)₂
as well as other groups such as
-CONH₂
-CONHCH₃
-OCOCH₃
-N(COCH₃)H
-N(COCH₃)C₁₋₃-alkyl
-N(COCH₃)-mono, bis or tris-hydroxy C₁₋₄-alkyl
-N(COCH₂OH)-mono, bis or tris-hydroxy C₁₋₄-alkyl
-C(COCH₃)(mono, bis or tris-hydroxy C₁₋₄-alkyl)₂
-N(COCH₂OH)₂
-CON(CH₂CHOHCH₂OH) (CH₂CH₂OH)
-CONH-C(CH₂OH)₃ and
-CONH-CH(CH₂OH) (CHOHCH₂OH).

In general, the ring substituent groups will preferably each comprise a polyhydroxy C₁₋₄-alkyl group, such as 1,3-dihydroxyprop-2-yl or 2,3-dihydroxyprop-1-yl.

Other such ring substituent groups as are conventional within the field of triiodophenyl X-ray contrast agents may also be used.

Where present, X may be a bond or a 1-7, e.g. 1,2,3 or 4 member atom chain comprising carbon, nitrogen, oxygen or sulphur atoms, e.g. a bond, an O, S, N or CO atom chain, an NCN, OCN, CNC, OCO, NSN, CSN, COC, OCC or CCC atom chain. Examples include: an oxygen atom; a group NR⁶, CO, SO₂ or CR⁶₂, a group COCO, CONR⁶, COCR₂⁶, SOCR₂⁶, SO₂NR⁶, CR⁶₂CR⁶₂, CR⁶₂NR⁶ or CR⁶₂O group, a group NR⁶CONR⁶, OCONR⁶, CONR⁶CO, CoNR⁶CR⁶₂, OCCO, CR⁶₂OCR⁶₂, OCR⁶₂CO, CR⁶₂CONR⁶, CR⁶₂CR⁶₂CR⁶₂, COCR⁶₂CO, CR⁶₂NR⁶CR⁶₂, CR⁶₂SO₂NR⁶, CR⁶₂OCO and NR⁶SO₂NR⁶ groups (where R⁶ is hydrogen or C₁₋₆ alkyl or alkoxy optionally substituted by hydroxy, alkoxy, oxa or oxo (e.g. a polyhydroxyalkyl, formyl, acetyl, hydroxy, alkoxy or hydroxyalkyl group, or where attached to a carbon R⁶ may also be a hydroxy group). When X provides a 4-7 atom linkage, conventional linker groups, such as for example those suggested by WO-93/10078, US-A-4348377 or WO-94/14478 may be used.

In general such linkages will comprise optionally aza or oxa substituted alkylene chains optionally carrying R⁶ substituents, especially such groups terminating with imine nitrogen or, more preferably, carbonyl carbon atoms, preferably belonging to iminocarbonyl functional units within the chain. Hydroxylated chains, such as are found in iodixanol are particularly preferred.

Examples of such chains are NCCN, NCCCN, CNCCCNC, and CNCCN, e.g.
-NR⁶COCONR⁶-
-NR⁶COCR⁶₂CONR⁶-
-NR⁶CR⁶₂CR⁶OHCR⁶₂NR⁶-
-CONR⁶CR⁶₂CONR⁶⁻ and
-N(COR⁶)CR⁶₂CR⁶OHN(COR⁶)-,
e.g. as found in iotrolan, iofratol, ioxaglic acid and iodixanol, or as otherwise indicated in WO-94/14478.

The process of the invention is preferably carried out in a multi-compartment (e.g. 2 or 3 compartment) reaction vessel with porous inter-compartment barriers, for example of glass frit or a permeable membrane material such as Nafion. The anode and the cathode are then preferably disposed in separate compartments and in this way the anodic compartment may be used as the reaction chamber. The aromatic compound which is intended to be iodinated can be added into the reaction vessel as a whole or into the anodic chamber only. Addition can be before, during or after the electrochemical generation of the I⁺ species.

The anode itself, the working electrode, is preferably of carbon (e.g. graphitic or vitreous carbon), conductive titanium dioxide, platinum or a platinum alloy (e.g. Pt/Ir/Ti). For metal or metal alloy anodes, the anode is conveniently in sheet, plate or gauze form. Platinum gauze and graphitic carbon anodes are preferred.

The nature of the cathode is less important and conventional cathodic materials such as carbon, platinum, palladium, lead, copper and stainless steel or mixtures thereof may be used.

The solvent used is water, optionally together with one or more water-miscible organic co-solvents, preferably a polar and/or protic solvent such as an alkanol (e.g. methanol, ethanol, propanol, iso-propanol or n-butanol), an ether (e.g. a cyclic ether such as tetrahydrofuran), a ketone (such as acetone), a carboxylic acid (such as acetic or trifluoroacetic acid), dimethylformamide or dimethylsulphoxide, or acetonitrile. Acetonitrile or an alkanol such as methanol is generally preferred. Aromatic organic solvents such as toluene should of course not generally be used.

The water to organic co-solvent ratio is preferably 1:3 to 3:1 by weight.

The acidic nature of the solvent system may be achieved by selection of an acidic organic co-solvent such as acetic acid or trifluoroacetic acid or may be by virtue of the inclusion of a strong organic or inorganic acid such as sulphuric or fluroboric acid.

The pH of the solvent system is preferably below 7, particularly 0 to 3, especially 1 to 2.

For the electrochemical generation of I⁺ to be effective, it is preferred that the solvent system should include an electrolyte species so as to enhance solvent conductivity. The concentration of electrolyte used should be sufficient to give adequate conductivity, for example 1-100% by weight relative to the non-ionic solvents, especially 1-10%. The electrolyte should not of course provide a competing species in the anode compartment and in general fluoroborates, sulphates and perchlorates are preferred, for example lithium perchlorate, tetraethylammonium perchlorate, tetrabutylammonium fluoroborate, tetra-n-propylammoniumfluoroborate, NaBF₄, NaBF₄, (CH₃)₄NBF₄, Na₂SO₄ and (Bu)₄NBF₄, especially lithium perchlorate, NaBF₄, (CH₃)₄NBF₄ or (Bu)₄NBF₄.

The iodine source used in the process of the present invention may be introduced into the reaction mixture either as a whole or simply into the anodic compartment of the compartmented reaction vessel. For this purpose, iodine or an iodide (such as NaI, KI, HI or an alkylammoniumiodide) may be used. Iodine, KI and NaI are preferred.

The iodine source will preferably be used at a concentration sufficient to provide 100-150% of iodine atoms relative to the unsubstituted ringsites on the target aromatic species. In general, the target compounds will be used at concentrations of from about 0.1 to 1M, and preferred iodine concentrations are thus 0.15 to 2.25M.

In the reaction, a voltage is applied across the cathode and anode to generate the I⁺ species. This voltage is preferably 1-50V, especially 5-30V.

During the process of the invention, the reaction mixture is preferably cooled, for example by immersion of the reaction vessel in a water bath or by provision of the reaction vessel with a water jacket so as to maintain the solvent temperature below 25°C.

Following the process of the invention, the iodinated product may be isolated by removal of the organic solvent followed by filtration or chromatography.

The process of the invention is illustrated further by the-following non-limiting examples:

### EXAMPLE 1

### Procedure A for the electrochemical iodination

In a water-jacketed cell containing a platinum anode (4x5cm), a magnetic stirring bar, a cathode compartment separated by a glass frit and a stainless steel cathode (5cm²) was placed 80 ml of a 0.15 M NaBF₄ solution in methanol/water (1/1). Iodine (0.76g, 3.0 mmol) was dissolved in the anolyte and, after adjustment of the pH to 1.5 with aqueous HBF₄, a constant current of 200mA was passed through the solution while the temperature was kept at 20°C. After passage of 3F/mol I₂, the anolyte solution was used for iodination.

### EXAMPLE 2

### 3,5-Bis (2.3-dihydroxypropylaminocarbonyl)-2,4,6-triiodo-aniline

### Procedure B for the electrochemical iodination (substrate present)

In a water-jacketed cell containing a platinum anode (4x5cm), a magnetic stirring bar, a cathode compartment separated by a glass frit and a stainless steel cathode (5cm²) was placed 80 ml of a 0.15 M NaBF₄ solution in methanol/water (1/1). Iodine (0.76g 0.3mmol) and 3,5-bis(2,3-dihydroxypropylaminocarbonyl)aniline (0.33g, 1mmol) was dissolved in the anolyte and, after adjustment of the pH to 1.5 with aqueous HBF₄, a constant current of 200 mA was passed through the solution while the temperature was kept at 20°C. After passage of 3F/mol I₂, the anolyte was stirred at 60°C for 24h. Analysis by HPLC indicated a quantitative conversion of the substrate into 3,5-bis(2,3-dihydroxypropylaminocarbonyl)-2,4,6-triodoaniline.

### EXAMPLE 3:3,5-Bis(2,3-dihydroxypropylaminocarbonyl)-2,4,6-triodoaniline

To the solution obtained according to Procedure A (Example 1) was added 3,5-bis(2,3-dihydroxypropylaminocarbonyl)-aniline (0.33g, lmmol) dissolved in water (40ml). After adjustment of the pH to 1.1 with aqueous HBF₄, the solution was heated to 80°C. HPLC analysis after 2.5 hours indicated 99.8% conversion to the triiodinated product. Purification by preparative HPLC gave the title product as a white crystalline material (72% isolated yield).

### EXAMPLE 4: 3-Hydroxymethyl-5 (2,3-dihydroxypropylaminocarbonyl)-2,4,6-triodoaniline

To a iodination solution prepared according to Procedure A (Example 1) from 9.9g of I₂ in 400ml solvent was added 3-hydroxymethyl-5-(2,3-dihydroxypropylaminocarbonyl)-aniline (5g, 20.8mmol) dissolved in water (100ml). The solution was stirred at 60°C for 8h. After adjustment of the pH to 5 with aqueous NaOH, methanol was removed by evaporation and the residue was lyophilized. The solid crude product was then redissolved in CH₂Cl₂/MeOH (7:3, v/v) and filtered through a pad of silica gel. Evaporation gave 10g (78%) of the title product as a white solid. ¹H NMR(300MHz, CD₃OD): 5.21(s, 2H), 3.90-3.98(m, 1H), 3.22-3.77(m,4H), 2.14(s,2H).

### EXAMPLE 5

### 3,5-Bis(2,3-dihydroxypropylaminocarbonyl)-2,4,6-triodoaniline

### Procedure C for electrochemical iodination (starting from KI)

In a water-jacketed cell containing a platinum anode (4x5 cm), a magnetic stirring bar, a cathode compartment separated by a glass frit and a stainless steel cathode (5 cm²) was placed 40 ml of a 0.25 M NaPF₆ solution in methanol/water(1/1) containing KI (0.498 g, 3 mm0l). After adjustment of the pH to ca 1.6 using HPF₆, a constant current of 200 mA was passed through the solution until 6F/mol I⁻ had been consumed. A solution of 3,5-bis(2,3-dihydroxypropylaminocarbonyl)-aniline (0.164 g, 0.5 mmol) in water (20 ml) was added and the mixture was stirred at 40 °C for 96 h. Analysis by HPLC indicated a 65% conversion of the starting material into 3,5-bis(2,3-dihydroxypropylaminocarbonyl)2,4,6-triiodoaniline.

### EXAMPLE 6

### 3-(2,3-Dihydroxypropylaminocarbonyl)-2,4,6-triiodo-5-(3'-amino-2',4',6'-triiodo-5'(2,3-dihydroxypropylaminocarbonyl)-benzoylamino)-aniline

To an iodination solution prepared according to Procedure A (Example 1) from I₂ (0.99 g,3.9 mmol) in a mixture of methanol (30 ml) and water (10 ml) containing NaBF₄ (0.2 M) was added 3-(2,3-dihydroxypropyl-aminocarbonyl)-5-(3'-amino-5'-(2,3-dihydroxypropylaminocarbonyl)-benzoylamino)-aniline (0.66 g, 1.04 mmol) dissolved in a mixture of methanol and water (5 ml, 3:1). The solution was stirred for at 40°C for 48 hours. After addition of aqueous NaHSO₃ and neutralisation with aqueous NaHCO₃, the solvents were evaporated. HPLC analysis of the residue indicated a greater than 90% conversion to the desired product.

### EXAMPLE 7

### 3-Hydroxymethyl-5-[1,3,4-trihydroxybut-2-ylamino-carbonyl]-2,4,6-triiodoaniline

To a iodination solution prepared according to Procedure A from 10.6 g of I₂ in 625 ml solvent (0.2 M NaBF₄ in methanol/water 3:1) was added 3-hydroxymethyl-5-[1,3,4-trihydroxybut-2-ylamino-carbonyl]-2,4,6-aniline (5 g, 18.5 mmol) dissolved in water (25 ml). The solution was stirred at 60°C for 5-6 hours. After cooling, the solution was decolorized by addition of a saturated aqueous solution of sodium bisulfite (5 ml). The pH was adjusted to 5 with aqueous NaOH, methanol was removed by evaporation and the residue was lyophilized. The solid crude product was then redissolved in CH₂Cl₂/MeOH (4:1, v/v) and filtered through a pad of silica gel. Evaporation gave 8.9 g (74%) of the product as a white crystalline solid.

¹H NMR (300 MHz, DMSO-d₆): 7.61-7.69 (m, 1H), 5.42 (br s, 2H), 5.04 (br s, 1H), 4.87 (d, J=6.0 Hz, 2H), 4.68 (br s, 1H), 4.45 (br s, 2H), 3.82-3.95 (m, 3H), 3.63-3.73 (m, 2H), 3.46-3.54 (m, 1H).

¹³C NMR (75 MHz, DMSO-d₆): 170.6, 149.7, 148.3, 145.1, 101.6, 100.5, 96.7, 87.6, 82.2, 81.7, 80.0, 79.8, 75.0, 69.5, 63.9, 59.2, 53.3, 49.0.

### EXAMPLE 8 (COMPARATIVE)

The title compound of Example 4 was prepared by the conventional triiodination process as follows:
3-hydroxymethyl-5-(2,3-dihydroxypropylaminocarbonyl)-aniline (500 mg, 2.1 mmol) was dissolved in water (175 ml) and an aqueous solution of KICl₂ (70% w/w) was added in portions of 0.1 ml during 8 hours. A total amount of 1.0 ml KICl₂ solution was added. After a total reaction time of 6 hours, the solution was extracted with ethyl acetate (1000 ml) which was separated and washed with an aqueous solution of Na₂S₂O₃ (0.2 M, 100 ml). Evaporation followed by purification by preparative HPLC gave the pure product.

### EXAMPLE 9 (COMPARATIVE)

The title compound of Example 6 was prepared by the conventional triiodination process as follows:
3-(2,3-dihydroxypropylaminocarbonyl)-5-(3'-amino-5'-(2,3-dihydroxypropylaminocarbonyl)-benzoylamino)-aniline (2.0 g) was dissolved in a mixture of methanol and water (1:3, 1200 ml) and an aqueous solution of KICl₂ (5.34 g, 22.6 mmol) was added. The reaction mixture was stirred at 40°C for 30 hours and then a 0.5 M aqueous solution of NaHSO₃ (1 ml) was added. After evaporation of the solvents, the residue was purified by preparative HPLC.

### EXAMPLE 10

### Comparison of yields from the conventional process and the process according to the invention

| Compound | Conventional Process (% Yield) | Electroiodination Process of the Invention (% Yield) |
|---|---|---|
| Example 4/8 | 36 | 78 |
| Example 6/9 | 14 | 25-30 |

## Claims

1. A process for the preparation of a 2,4,6-triiodinated or 2,4,6,2',4',6'-hexaiodinated 3,5-disubstituted-aniline or 3,3'-disubstituted-5,5'-linked bisaniline, which process comprises electrochemically iodinating a 3,5-disubstituted-aniline or a 3,3'-disubstituted-5,5'-linked bisaniline in an aqueous acidic solvent.

2. A process as claimed in claim 1 wherein said acidic solvent comprises at least one water-miscible organic solvent.

3. A process as claimed in claim 1 for the preparation of compounds of formula I 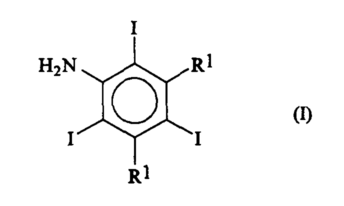 (wherein each R¹ independently represents a hydrophilic group or one R¹ group represents a hydrophilic group and the other R¹ group may represent a group of formula II 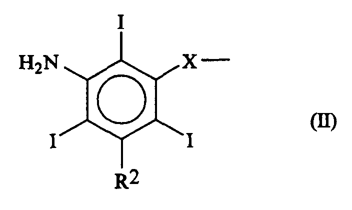 where R² is as defined for R¹ with the exception of the group of formula II and X is a bond or a moiety providing a 1-7 atom chain linking the aromatic rings), which process comprises electrochemically iodinating a compound of formula III 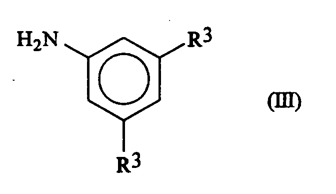 (wherein each R³ independently represents a hydrophilic group or one R³ group represents a hydrophilic group and the other R³ group represents a group of formula IV 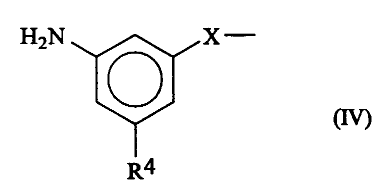 where R⁴ is as described for R³ with the exception of the group of formula IV).

4. A process as claimed in claim 3 wherein in formula I, II, III and IV hydrophilic R¹, R², R³ or R⁴ groups are straight chain or branched C₁₋₈-alkyl groups with one or more CH₂ or CH moieties replaced by oxygen or nitrogen atoms or substituted by one or more groups selected from oxo, hydroxy, alkoxy, amino, carboxyl, carboxyl derivative, and oxo substituted sulphur and phosphorus atoms.

5. A process as claimed in claim 3 wherein in formula I, II, III and IV hydrophilic R¹, R², R³ or R⁴ groups are polyhydroxyalkyl, hydroxyalkoxyalkyl and hydroxypolyalkoxyalkyl optionally attached to the phenyl group via an amide, CO, SO or SO₂ linkage.

6. A process as claimed in claim 3 wherein in formula I, II, III and IV hydrophilic R¹, R², R³ or R⁴ groups are hydroxyalkylaminocarbonyl, N-alkylhydroxyalkylaminocarbonyl, bis-hydroxyalkylaminocarbonyl, COCH₂OH or SO₂CH₂OH groups.

7. A process as claimed in claim 3 wherein in formula I, II, III and IV hydrophilic R¹, R², R³ or R⁴ groups are
-CONH-CH₂CH₂OH,
-CONH-CH₂CHOHCH₂OH,
-CONH-CH(CH₂OH)₂,
-CON(CH₂CH₂OH)₂,
-CONH₂,
-CONHCH₃,
-OCOCH₃,
-N(COCH₃)H,
-N(COCH₃)C₁₋₃-alkyl,
-N(COCH₃)-mono, bis or tris-hydroxy C₁₋₄-alkyl,
-N(COCH₂OH)-mono, bis or tris-hydroxy C₁₋₄-alkyl,
-C(COCH₃) (mono, bis or tris-hydroxy C₁₋₄-alkyl)₂,
-N(COCH₂OH)₂,
-CON(CH₂CHOHCH₂OH) (CH₂CH₂OH),
-CONH-C(CH₂OH)₃ or
-CONH-CH(CH₂OH)(CHOHCH₂OH) groups.

8. A process as claimed in claim 3 wherein in the compound of formula III each aminophenyl ring is substituted by at least one polyhydroxy C₁₋₄-alkyl group.

9. A process as claimed in claim 3 wherein in the compound of formula III X is a bond, an oxygen atom, or a group NR⁶, CO, SO₂, CR⁶₂, COCO, CONR⁶, COCR₂⁶, SOCR₂⁶, SO₂NR⁶, CR⁶₂CR⁶₂, CR⁶₂NR⁶, CR⁶₂O, NR⁶CONR⁶, OCONR⁶, CONR⁶CO, CONR⁶CR⁶₂, OCCO, CR⁶₂OCR⁶₂, OCR⁶₂CO, CR⁶₂CONR⁶, CR⁶₂CR⁶₂CR⁶₂, COCR⁶₂CO, CR⁶₂NR⁶CR⁶₂, CR⁶₂SO₂NR⁶, CR⁶₂OCO or NR⁶SO₂NR⁶ (where R⁶ is hydrogen or C₁₋₆ alkyl or alkoxy optionally substituted by hydroxy, alkoxy, oxa or oxo group).

10. A process as claimed in claim 3 wherein said compound of formula III is 3,5-tris(2,3-dihydroxypropylaminocarbonyl)aniline.

11. A process as claimed in claim 3 wherein said compound of formula III is 3-hydroxymethyl-5-(2,3-dihydroxypropylaminocarbonyl)aniline.

12. A process as claimed in claim 3 wherein said compound of formula III is 3-(2,3-dihydroxypropylaminocarbonyl)5-(3'-amino-5'-(2,3-dihydroxypropylaminocarbonyl)-benzoylamino)aniline.

13. A process as claimed in claim 1 wherein said electroiodination is effected in a multi-compartment reaction vessel with porous inter-compartment barriers, with an anode and a cathode disposed in separate compartments in said vessel.

14. A process as claimed in claim 1 wherein said electroiodination is effected using an anode of carbon, titanium dioxide, platinum or a platinum alloy.

15. A process as claimed in claim 2 wherein said organic solvent is a polar or protic solvent.

16. A process as claimed in claim 15 wherein said organic solvent is selected from the group consisting of alkanols, ethers, ketones, carboxylic acids, dimethylformamide, dimethylsulphoxide and acetonitrile.

17. A process as claimed in claim 1 wherein said acidic solvent contains a fluoroborate, sulphate or perchlorate electrolyte.

18. A process as claimed in claim 1 further comprising the step of isolating the iodinated product.

## Patentansprüche

1. Verfahren zur Herstellung eines 2,4,6-triiodierten oder 2,4,6,2',4',6'-hexaiodierten 3,5-disubstituierten Anilins oder 3,3'-disubstituierten-5,5'-verknüpften Bisanilins, umfassend die elektrochemische Iodierung eines 3,5-disubstituierten Anilins oder eines 3,3'-disubstituierten-5,5'-verknüpften Bisanilins in einem wässrigen sauren Lösungsmittel.

2. Verfahren nach Anspruch 1, wobei das saure Lösungsmittel wenigstens ein wassermischbares organisches Lösungsmittel umfasst.

3. Verfahren nach Anspruch 1 zur Herstellung einer Verbindung der Formel I 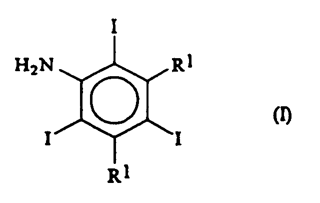 wobei jeder der Reste R¹ unabhängig voneinander für eine hydrophile Gruppe steht, oder einer der Reste R¹ für eine hydrophile Gruppe und der andere für eine Gruppe der Formel II 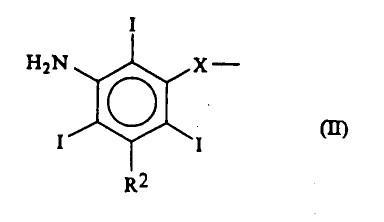 steht, worin R² dieselben Bedeutungen wie R¹ mit der Ausnahme der Gruppe der Formel II besitzt und X² für eine Bindung oder für eine Einheit steht, welche die aromatischen Ringe mit einer 1-7 atomigen Kette verbindet, und wobei das Verfahren die elektrochemische Iodierung einer Verbindung der Formel III 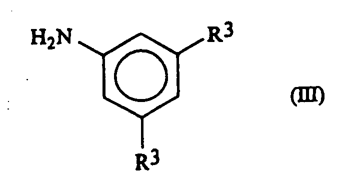 umfasst, wobei jeder der Reste R³ unabhängig voneinander für eine hydrophile Gruppe steht oder einer der Reste R³ für eine hydrophile Gruppe und der andere für eine Gruppe der Formel IV 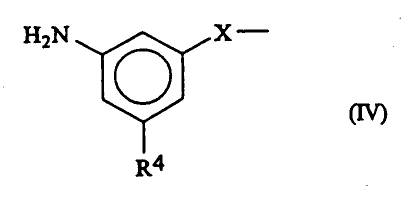 steht, worin R⁴ dieselben Bedeutungen wie R³ mit der Ausnahme der Gruppe der Formel IV besitzt.

4. Verfahren nach Anspruch 3 wobei die hydrophilen Gruppen R¹, R², R³ oder R⁴ in den Formeln I, II, III und IV geradkettige oder verzweigte C₁₋₈-Alkylgruppen sind, wobei eine oder mehrere CH₂- oder CH-Einheiten durch Sauerstoff- oder Stickstoffatome ersetzt oder durch eine oder durch mehrere Gruppen, ausgewählt unter Oxo-, Hydroxy-, Alkoxy-, Amino-, Carboxyl- und Carbonylderivaten und oxo-substituierten Schwefel- und Phosphoratomen substituiert sind.

5. Verfahren nach Anspruch 3, wobei die hydrophilen Gruppen R¹, R², R³ oder R⁴ in den Formeln I, II, III und IV für Polyhydroxyalkyl, Hydroxyalkoxyalkyl und Hydroxypolyalkoxyalkyl stehen, welche gegebenenfalls über eine Amid-, CO-, SO- oder SO₂-Bindung an die Phenylgruppe gebunden sind.

6. Verfahren nach Anspruch 3, wobei die hydrophilen Gruppen R¹, R², R³ und R⁴ in den Formeln I, II, III und IV Hydroxyalkylaminocarbonyl-, N-alkylhydroxyalkylaminocarbonyl-, Bishydroxyalkylaminocarbonyl-, COCH₂OH- oder SO₂CH₂OH-Gruppen sind.

7. Verfahren nach Anspruch 3, wobei die hydrophilen Gruppen R¹, R², R³ oder R⁴ in den Formel I, II, III und IV für die Gruppen
-CONH-CH₂CH₂OH,
- CONH-CH₂CHOHCH₂OH,
-CONH-CH(CH₂OH)₂,
-CON(CH₂CH₂OH)₂,
-CONH₂,
-CONHCH₃
-OCOCH₃,
-N(COCH₃)H,
-N(COCH₃)C₁₋₃-alkyl,
-N(COCH₃)-mono-, -bis- oder -tris-hydroxy-C₁₋₄-alkyl,
-N(COCH₂OH)-mono, -bis- oder -tris-hydroxy-C₁₋₄-alkyl,
-C(COCH₃) (mono-, bis- oder tris-hydroxy-C₁₋₄-alkyl)₂,
-N(COCH₂OH)₂,
-CON(CH₂CHOHCH₂OH) (CH₂CH₂OH),
-CONH-C(CH₂OH)₃ oder
-CONH-CH(CH₂OH) (CHOHCH₂OH)
stehen.

8. Verfahren nach Anspruch 3, wobei in der Verbindung der Formel III jeder Aminophenylring durch wenigstens eine Polyhydroxy-C₁₋₄-alkylgruppe substituiert ist.

9. Verfahren nach Anspruch 3, wobei in der Formel III X für eine Bindung, ein Sauerstoffatom oder eine NR⁶-, CO-, SO₂-, CR⁶₂-, COCO-, CONR⁶-, COCR⁶₂-, SOCR⁶₂-, SO₂NR⁶-, CR⁶₂CR⁶₂-, CR⁶₂NR⁶-, CR⁶₂O-, NR⁶CONR⁶-, OCONR⁶-, CONR⁶CO-, CONR⁶CR⁶₂-, OCCO-, CR⁶₂OCR⁶₂-, OCR⁶₂CO-, CR⁶₂CONR⁶-, CR⁶₂CR⁶₂CR⁶₂-, COCR⁶₂CO-, CR⁶₂NR⁶CR⁶₂-_{,} CR⁶₂SO₂ NR⁶-, CR⁶₂OCO- oder NR⁶SO₂NR⁶-Gruppe steht, wobei R⁶ für Wasserstoff oder C₁₋₆-Alkyl oder -Alkoxy steht, welches gegebenenfalls durch Hydroxy, Alkoxy, Oxa oder Oxo substituiert ist.

10. Verfahren nach Anspruch 3, wobei die Verbindung der Formel III 3,5-Tris(2,3-dihydroxypropylaminocarbonyl)anilin ist.

11. Verfahren nach Anspruch 3, wobei die Verbindung der Formel III 3-Hydroxymethyl-5-(2,3-dihydroxypropylaminocarbonyl)-anilin ist.

12. Verfahren nach Anspruch 3, wobei die Verbindung der Formel III 3-(2,3-Dihydroxypropylaminocarbonyl)5-(3'-amino-5'-(2,3-dihydroxypropylaminocarbonyl)benzoylamino)anilin ist.

13. Verfahren nach Anspruch 1, wobei die Elektroiodierung in einem mehrzelligen Reaktionsgefäß mit porösen Zwischenwänden und einer Anode und einer Kathode, die in separaten Zellen angeordnet sind, durchgeführt wird.

14. Verfahren nach Anspruch 1, wobei die Elektroiodierung unter Einsatz einer Kohlenstoff-, Titandioxid-, Platin- oder Platinlegierungs-Anode durchgeführt wird.

15. Verfahren nach Anspruch 2, wobei das organische Lösungsmittel ein polares oder protisches Lösungsmittel ist.

16. Verfahren nach Anspruch 15, wobei das organische Lösungsmittel ausgewählt ist unter Alkanolen, Ethern, Ketonen, Carbonsäuren, Dimethylformamid, Dimethylsulfoxid und Acetonitril.

17. Verfahren nach Anspruch 1, wobei das saure Lösungsmittel einen Fluorborat-, Sulfat- oder Perchlorat-Elektrolyt enthält.

18. Verfahren nach Anspruch 1, welches zusätzlich den Schritt der Isolierung des iodierten Produktes umfasst.

## Revendications

1. Procédé de préparation d'une aniline 3,5-disubstituée ou d'une bis-aniline 3,3'-disubstituée et 5,5'-liée, 2,4,6-triiodée ou 2,4,6,2',4 ',6'-hexaiodée, selon lequel on iode électrochimiquement une aniline 3,5-disubstituée ou une bis-aniline 3,3'-disubstituée et 5,5'-liée dans un solvant acide aqueux.

2. Procédé selon la revendication 1, dans lequel ledit solvant acide comprend au moins un solvant organique miscible dans l'eau.

3. Procédé selon la revendication 1, pour la préparation de composés de formule I : 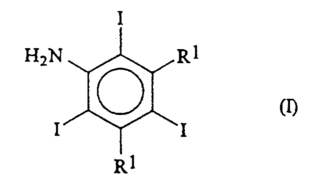 (dans laquelle chaque groupe R¹ représente indépendamment un groupe hydrophile, ou un groupe R¹ représente un groupe hydrophile et l'autre groupe R¹ peut représenter un groupe de formule II : 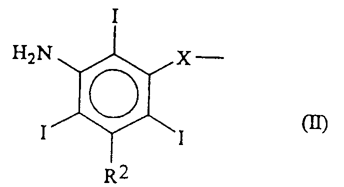 dans laquelle R² est tel que défini pour R¹, à l'exception du groupe de formule II, et X représente une liaison ou une partie formant une chaîne de 1 à 7 atomes liant les cycles aromatiques), selon lequel on iode électrochimiquement un composé de formule III : 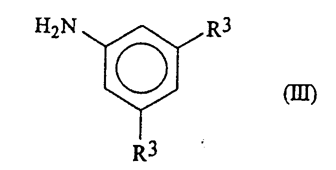 (dans laquelle chaque groupe R³ représente indépendamment un groupe hydrophile, ou un groupe R³ représente un groupe hydrophile et l'autre groupe R³ représente un groupe de formule IV : 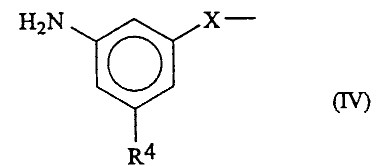 dans laquelle R⁴ est tel que décrit pour R³, à l'exception du groupe de formule IV).

4. Procédé selon la revendication 3, dans lequel dans les formules I, II, III et IV, les groupes hydrophiles R¹, R², R³ ou R⁴ sont des groupes alkyle en C₁-C₈ ramifiés ou à chaîne linéaire comportant une ou plusieurs parties CH₂ ou CH remplacées par des atomes d'oxygène ou d'azote ou substituées par un ou plusieurs groupes choisis parmi les groupes oxo, hydroxy, alkoxy, amino, carboxyle, dérivés de groupe carboxyle, et les atomes de soufre et de phosphore à substituant oxo.

5. Procédé selon la revendication 3, dans lequel dans les formules I, II, III et IV, les groupes hydrophiles R¹, R², R³ ou R⁴ représentent des groupes polyhydroxyalkyle, hydroxyalkoxyalkyle et hydroxypolyalkoxyalkyle éventuellement liés au groupe phényle par l'intermédiaire d'une liaison amide, CO, SO ou SO₂.

6. Procédé selon la revendication 3, dans lequel dans les formules I, II, III et IV, les groupes hydrophiles R¹, R², R³ ou R⁴ représentent des groupes hydroxyalkylaminocarbonyle, N-alkylhydroxyalkylaminocarbonyle, bis-hydroxyalkylaminocarbonyle, COCH₂OH ou SO₂CH₂OH.

7. Procédé selon la revendication 3, dans lequel dans les formules I, II, III et IV, les groupes hydrophiles R¹, R², R³ ou R⁴ représentent des groupes :
-CONH-CH₂CH₂OH,
-CONH-CH₂CHOHCH₂OH,
-CONH-CH(CH₂OH)₂,
-CON(CH₂CH₂OH)₂,
-CONH₂,
-CONHCH₃,
-OCOCH₃,
-N(COCH₃)H,
-N(COCH₃) (alkyle en C₁-C₃),
-N(COCH₃)-(mono, bis ou tris-hydroxyalkyle en C₁-C₄),
-N(COCH₂OH) - (mono, bis ou tris-hydroxyalkyle en C₁-C₄),
-C(COCH₃)(mono, bis ou tris-hydroxyalkyle en C₁-C₄),
-N(COCH₂OH)₂,
-CON(CH₂CHOHCH₂OH) (CH₂CH₂OH),
-CONH-C(CH₂OH)₃ ou
-CONH-CH(CH₂OH) (CHOHCH₂OH).

8. Procédé selon la revendication 3, dans lequel dans le composé de formule III, chaque cycle aminophényle est substitué par au moins un groupe polyhydroxyalkyle en C₁-C₄.

9. Procédé selon la revendication 3, dans lequel dans le composé de formule III, X représente une liaison, un atome d'oxygène, ou un groupe NR⁶, CO, SO₂ ou CR⁶₂, un groupe COCO, CONR⁶, COCR₂⁶, SOCR₂⁶, SO₂NR⁶, CR⁶₂CR⁶₂, CR⁶₂NR⁶ ou CR⁶₂O, un groupe NR⁶CONR⁶, OCONR⁶, CONR⁶CO, CONR⁶CR⁶₂, OCCO, CR⁶₂OCR⁶₂, OCR⁶₂CO, CR⁶₂CONR⁶, CR⁶₂CR⁶₂CR⁶₂, COCR⁶₂CO, CR⁶₂NR⁶CR⁶₂, CR⁶₂SO₂NR⁶, CR⁶₂OCO et NR⁶SO₂NR⁶ (dans lesquels R⁶ représente un atome d'hydrogène ou un groupe alkyle ou alkoxy en C₁-C₆ éventuellement substitué par un groupe hydroxy, alkoxy, oxa ou oxo).

10. Procédé selon la revendication 3, dans lequel ledit composé de formule III est la 3,5-tris(2,3-dihydroxypropylaminocarbonyl)aniline.

11. Procédé selon la revendication 3, dans lequel ledit composé de formule III est la 3-hydroxyméthyl-5-(2,3-dihydroxypropylaminocarbonyl)-aniline.

12. Procédé selon la revendication 3, dans lequel ledit composé de formule III est la 3-(2,3-dihydroxypropylaminocarbonyl)-5-(3'-amino-5'-(2,3-dihydroxypropylaminocarbonyl)-benzoylamino)aniline.

13. Procédé selon la revendication 1, dans lequel ladite électroiodation est effectuée dans un réacteur à plusieurs compartiments comportant des barrières inter-compartiment poreuses, une anode et une cathode étant disposées dans des compartiments séparés dans ledit réacteur.

14. Procédé selon la revendication 1, dans lequel ladite électroiodation est effectuée en utilisant une anode de carbone, de dioxyde de titane, de platine ou d'un alliage de platine.

15. Procédé selon la revendication 2, dans lequel ledit solvant organique est un solvant polaire ou protique.

16. Procédé selon la revendication 15, dans lequel ledit solvant organique est choisi parmi les alcanols, les éthers, les cétones, les acides carboxyliques, le diméthylformamide, le diméthylsulfoxyde et l'acétonitrile.

17. Procédé selon la revendication 1, dans lequel ledit solvant acide contient un électrolyte de type fluoroborate, sulfate ou perchlorate.

18. Procédé selon la revendication 1, comprenant en outre l'étape d'isolement du produit iodé.
